# EUROPEAN PATENT APPLICATION

(11) **EP 4 559 478 A1**
(43) Date of publication of application: **28.05.2025**
(21) Application number: 23843036.7
(22) Date of filing: 20.07.2023
(51) Int. Cl.: A61K 39/145, A61P 31/16, C12Q 1/68, C12Q 1/70, G01N 33/53

(54) **METHOD FOR REDUCING PYROGENIC ACTIVITY OF INACTIVATED WHOLE INFLUENZA VIRUS PARTICLE VACCINES**

(30) Priority: 21.07.2022 JP 2022116683
(71) Applicant: Denka Company Limited, Tokyo 103-8338 (JP)
(72) Inventor: GOTANDA, Takuma, Gosen-shi, Niigata 959-1695 (JP); MITSUMATA, Ryotaro, Gosen-shi, Niigata 959-1695 (JP)
(74) Representative: Hoffmann Eitle
(86) International application number: PCT/JP2023/026580
(87) International publication number: WO 2024/019113

(57) **Abstract**

It is provided a method for reducing pyrogenic activity in the preparation of an inactivated whole influenza virus particle vaccine using an embryonated chicken egg method. The method for reducing pyrogenic activity of an inactivated whole influenza virus particle vaccine in a method for preparing the vaccine using an embryonated chicken egg method comprises a step of reducing an avian-derived microRNA content in a virus solution containing a whole influenza virus particle collected from an embryonated chicken egg.

## Description

### Technical Field

The present invention relates to a method for reducing pyrogenic activity of inactivated whole influenza virus particle vaccines.

### Background Art

Influenza viruses belong to *Orthomyxoviridae* family and are classified into A, B, C, and D types based on the difference in antigenicity of nucleoprotein and matrix protein present inside viruses. Types A and B are prevalent in winter, and infection with the viruses causes acute respiratory infection, i.e., influenza. As a method for preventing influenza viral infection, influenza vaccination is known, and in Japan, split vaccines prepared by splitting virus particles by ether treatment are mainly used. The split vaccines are excellent in safety, but suffer from having low ability to induce antibodies in children with little influenza viral infection and vaccination histories and elderly persons with weakened immune functions.

Now, highly safe split vaccines are distributed, but before 1970s, inactivated whole particle vaccines of which infectiousness was eliminated by chemically treatment of influenza virus particles were used. It has been reported that the inactivated whole particle vaccines include viral genomes which activate innate immunity and therefore exhibit ability to induce antibodies higher than that of split vaccines in children and elderly persons (Non Patent Literatures 1 and 2). On the other hand, in the case of the inactivated whole particle vaccines, the frequency of occurrence of adverse effects such as pyrogenic reaction is high (Non Patent Literatures 3 and 4). Therefore, the inactivated whole particle vaccines were replaced by split vaccines in 1970s. Accordingly, recently, a reduction in adverse effect of inactivated whole particle vaccines by a method other than split treatment has been studied, and an attempt of creating highly safe inactivated whole particle vaccines has been reported. Patent Literature 1 discloses that pyrogenic reaction can be suppressed by fixing virus particles with an aldehyde or the like. However, in the chemical treatment with an aldehyde or the like, the aldehyde could crosslink an active component, i.e., hemagglutinin (HA), impairing the intrinsic ability to induce antibodies of the inactivated whole particle vaccine.

The present applicant has disclosed in Patent Literature 2 that inactivated whole influenza virus particle vaccines include embryonated chicken egg-derived extracellular vesicles, the content of these extracellular vesicles can be reduced by hypotonic treatment during the process of preparing the whole particle vaccines, and vaccines with high ability to induce antibodies and attenuated pyrogenic activity can be obtained. However, since the molecule in the extracellular vesicle which affects the ability to induce antibodies and pyrogenic activity has not been specified and the pyrogen cannot be controlled during the manufacturing process, it was difficult to control the quality of a "vaccine with reduced pyrogenic activity".

### Citation List

### Patent Literature

Patent Literature 1: WO 2017/122635
Patent Literature 2: JP-A-2020-50604

### Non Patent Literature

Non Patent Literature 1: Gross P.A., Ennis F.A., Gaerlan P.F., Denson L.J., Denning C.R., and Schiffman D., A controlled double-blind comparison of reactogenicity, immunogenicity, and protective efficacy of whole-virus and split-product influenza vaccines in children. J Infect. Dis. 1977 Nov;136(5):623-32.
Non Patent Literature 2: McElhaney J.E., Meneilly G.S., Lechelt K.E., Beattie B.L., and Bleackley R.C., Antibody response to whole-virus and split-virus influenza vaccines in successful ageing. Vaccine. 1993;11(10):1055-60.
Non Patent Literature 3: Marine, W.M., et al., Reactions and serologic response in young children and infants after administration of inactivated monovalent influenza A vaccine. J. Pediatr. 1976 Jan;88(1):26-30
Non Patent Literature 4: Wright, P.F., et al., Clinical reactions and serologic response following inactivated monovalent influenza type B vaccine in young children and infants. J. Pediatr. 1976 Jan;88(1):31-35

### Summary of Invention

### Technical Problem

The present invention relates to providing a method for reducing pyrogenic activity in the preparation of an inactivated whole influenza virus particle vaccine using an embryonated chicken egg method.

### Solution to Problem

The present inventors have intensively studied, and as a result, have found that an inactivated whole particle vaccine prepared using an embryonated chicken egg method contains many microRNAs derived from the egg, some of the microRNAs have high sequence similarity with mammalian microRNAs that promote immune and inflammatory responses and some of the microRNAs also increase the pyrogenic activity of the inactivated whole particle vaccine, and the pyrogenic activity of the vaccine can be reduced by reducing the content of these microRNAs.

That is, the present invention relates to the following 1) to 5):
1) A method for reducing pyrogenic activity of an inactivated whole influenza virus particle vaccine in a method for preparing the vaccine using an embryonated chicken egg method, comprising a step of reducing an avian-derived microRNA content in a virus solution containing a whole influenza virus particle collected from an embryonated chicken egg.
2) The method according to 1), wherein the contents of at least gga-miR-22-3p and gga-miR-29b-1-5p are reduced;
3) An influenza virus vaccine according to 1) or 2), wherein the whole influenza virus particle contains one or both of a type A influenza virus strain and a type B influenza virus strain.
4) A method for evaluating pyrogenic activity of an inactivated whole influenza virus particle vaccine using an embryonated chicken egg method, comprising a step of measuring the contents of gga-miR-22-3p and gga-miR-29b-1-5p in the vaccine.
5) An inactivated whole influenza virus particle vaccine prepared by an embryonated chicken egg method and having reduced contents of gga-miR-22-3p and gga-miR-29b-1-5p.

### Advantageous Effects of Invention

According to the present invention, it is possible to provide a highly safe inactivated whole influenza virus particle vaccine by controlling the content of microRNAs which affect pyrogenic activity.

### Brief Description of Drawings

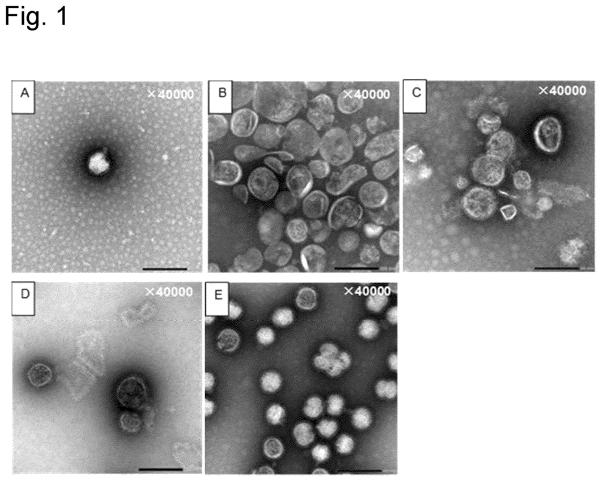

Figure 1 shows electron microscopic observation images of extracellular vesicles isolated from inactivated whole influenza virus particle vaccine, A: extracellular vesicle 1 (EV1) (×40,000 magnification), B: extracellular vesicle 2 (EV2) (×40,000 magnification), C: extracellular vesicle 3 (EV3) (×40,000 magnification), D: extracellular vesicle 4 (EV4) (×40,000 magnification), and E: virus particles (×40,000 magnification).

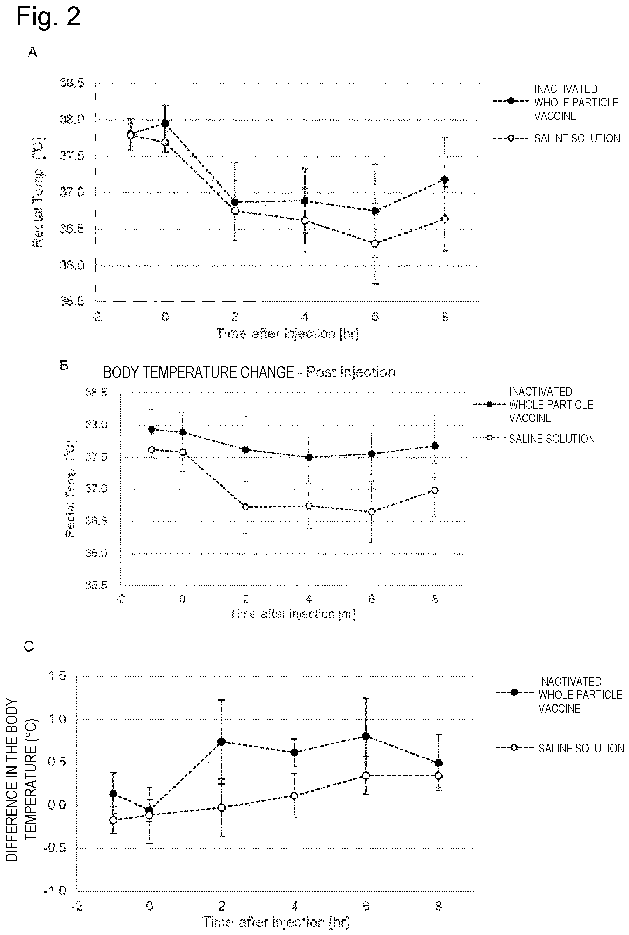

Figure 2 shows rectal temperature changes and difference in the body temperature changes in a pyrogenicity test of an inactivated whole influenza virus particle vaccine using mice, A: rectal temperature change - Baseline, B: rectal temperature change - Post injection, and C: difference in the body temperature change.

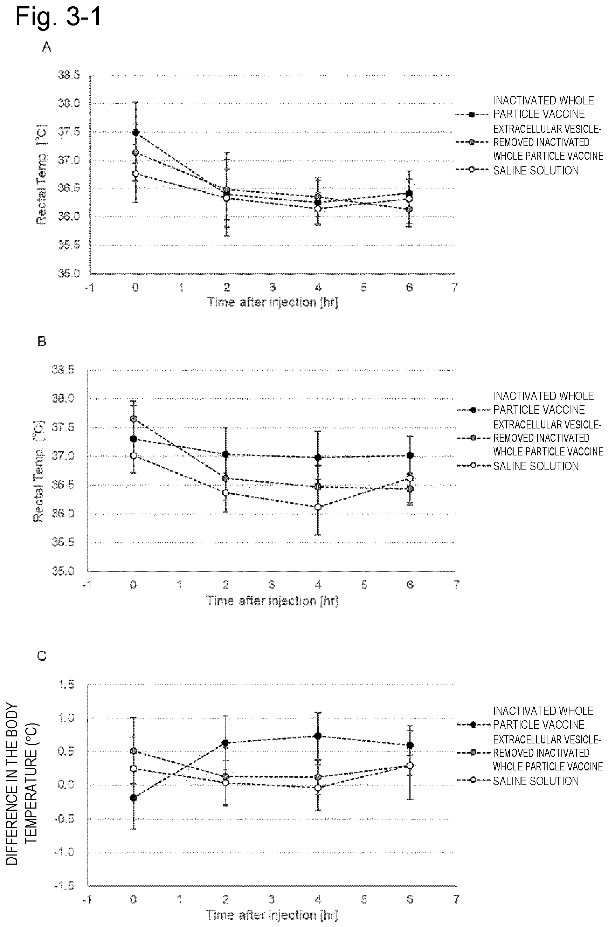

Figure 3-1 shows rectal temperature changes and difference in the body temperature changes in a pyrogenicity test of an extracellular vesicle-removed inactivated whole influenza virus particle vaccine, A: rectal temperature change - Baseline, B: rectal temperature change - Post injection, and C: difference in the body temperature change.

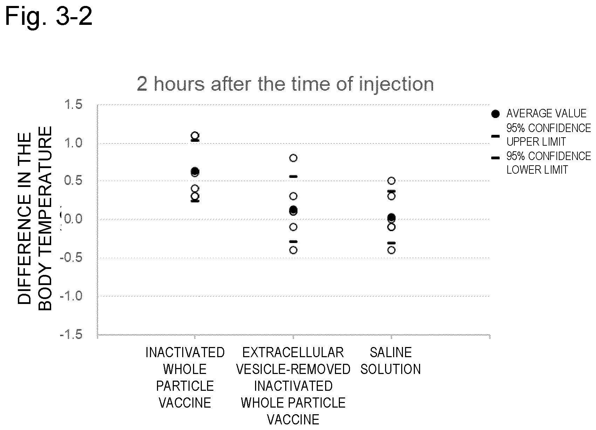

Figure 3-2 shows rectal temperature changes and difference in the body temperature changes in a pyrogenicity test of an extracellular vesicle-removed inactivated whole influenza virus particle vaccine (difference in the body temperature - at 2 hours after administration).

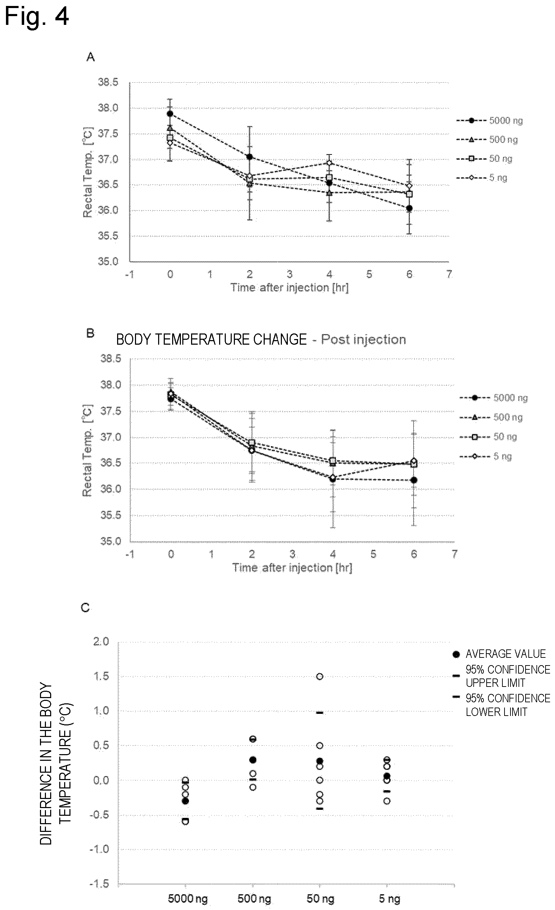

Figure 4 shows rectal temperature changes and difference in the body temperatures at 2 hours after administration in a microRNA administration dosage-setting test, A: rectal temperature change - Baseline, B: rectal temperature change - Post injection, and C: difference in the body temperature - at 2 hours after administration.

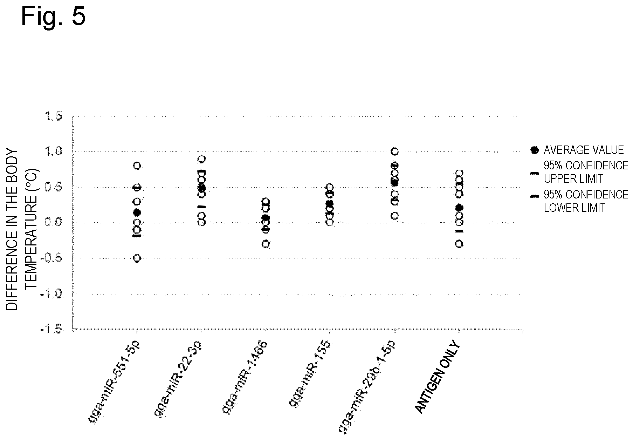

Figure 5 shows difference in the body temperatures at 2 hours after co-administration of each microRNA with an extracellular vesicle-removed inactivated whole particle vaccine.

### Description of Embodiments

The method for reducing pyrogenic activity of a vaccine of the present invention includes a step of reducing the avian-derived microRNA content in a virus solution containing a whole influenza virus particle collected from an embryonated chicken egg in a method for preparing an inactivated whole influenza virus particle vaccine using an embryonated chicken egg method.

As used herein, the term "influenza virus vaccine" means a vaccine containing at least either type A influenza virus or type B influenza virus antigen. That is, the influenza virus vaccine of the present invention may be a monovalent vaccine containing only type A influenza virus or type B influenza virus or a multivalent vaccine containing both.

The term "influenza virus" as used herein indicates type A influenza virus or type B influenza virus or both. The influenza virus includes all currently known subtypes, as well as any subtypes that may be isolated or identified in the future.

The influenza virus strain which is used in preparation of a vaccine of the present invention may be a strain isolated from an infected animal or patient or a recombinant virus established by genetic engineering in cultured cells.

As used herein, the term "influenza virus inactivated whole particle" means a virus particle retaining its viral form which is obtained by culturing the influenza virus and subjected to inactivation treatment, and the "inactivated whole influenza virus particle vaccine" indicates a vaccine of the inactivated virus particle.

The influenza virus inactivated whole particle of the present invention is prepared using an embryonated chicken egg method.

The "embryonated chicken egg method" is a method for inoculating a virus strain into an embryonated chicken egg and culturing it and then subjecting the virus suspension to clarification, concentration, purification, and inactivation to obtain a virus solution containing virus particles.

Here, the culturing is performed, after inoculation of an influenza virus strain, at 30°C to 37°C for about 1 to 7 days, preferably at 33°C to 35°C for about 2 days. After completion of the culturing, the virus suspension (infected allantoic fluid or infected cell culture supernatant) is collected and is subjected to centrifugation or filtration for clarification. Subsequently, for concentration, barium salt adsorption and elution reaction or ultrafiltration is performed. Virus purification can be performed using a means, for example, ultracentrifugation, such as sucrose density gradient centrifugation, or liquid chromatography.

The purified virus solution is inactivated. Examples of the method for inactivating the virus include formalin treatment, ultraviolet irradiation, and treatment with beta propiolactone, binary ethylenimine, or the like.

In the method of the present invention, the suspension of an influenza virus cultured in an embryonated chicken egg and collected is subjected to treatment for reducing the avian-derived microRNA content at any point during clarification, concentration, purification, and inactivation to obtain a vaccine.

In the present invention, the avian-derived microRNAs are microRNAs derived from the embryonated chicken egg used in the culture of a virus strain, and examples thereof include microRNAs that have high nucleotide sequence similarity with mammalian microRNAs and promotes an immune or inflammatory response in mammals. Specifically, the microRNAs are at least two types of microRNAs including gga-miR-29b-1-5p (SEQ ID NO: 1) and gga-miR-22-3p (SEQ ID NO: 2) or five types of microRNAs including gga-miR-1466 (SEQ ID NO: 3), gga-miR-155 (SEQ ID NO: 4), and gga-miR-551-5p (SEQ ID NO: 5) in addition to the above two.

The gga-miR-29b-1-5p has 96% sequence similarity with human hsa-miR-29b-1-5p (Accession No.: MIMAT0004514) and 88% sequence similarity with mouse mmu-miR-29b-1-5p (Accession No.: MIMAT0004523). The mmu-miR-29b-1-5p is known to have the function of positively regulating NIK/NF-κB signaling.

The gga-miR-22-3p has 100% sequence similarity with human hsa-miR-22-3p (Accession No.: MIMAT0000077). The hsa-miR-22-3p is known to have the function of positively controlling inflammatory response and the function of negatively regulating NLRP3 inflammasome.

As shown in Examples below, when gga-miR-22-3p or gga-miR-29b-1-5p and an extracellular vesicle-removed inactivated whole particle vaccine with confirmed reduced pyrogenic activity are co-administered to a mouse, the pyrogenic activity of the inactivated whole particle vaccine is increased. Accordingly, these microRNAs are believed to be pyrogens of an inactivated whole particle vaccine.

Examples of the method for reducing microRNAs include density gradient centrifugation, size exclusion chromatography, and immunoaffinity separation. Examples of the separation medium in the density gradient centrifugation include Iodexanol, Iohexol, Ficoll, Percoll, and cesium chloride. Examples of the antigen as a target in the immunoaffinity separation include CD9, CD63, and CD81.

For example, in the method using the density gradient centrifugation, an inactivated whole particle vaccine is overlaid on a 6% to 18% Iodexanol density gradient solution and is centrifugated at 100,600 × g at 4°C for 1.5 hours. After centrifugation, the supernatant is discarded such that the inactivated whole particle vaccine layer of the undermost layer remains, and 6.7 mM phosphate buffered saline (pH 7.2) is added thereto, followed by centrifugation at 141,400 × g at 4°C for 3 hours. After centrifugation, the supernatant is discarded, and the precipitate is suspended in 6.7 mM phosphate buffered saline (pH 7.2) to obtain an inactivated whole particle vaccine containing reduced microRNAs.

In the inactivated whole influenza virus particle vaccine with an avian-derived microRNA content reduced by the method of the present invention, for example, when 1 µg of the total RNA extracted from an inactivated whole particle vaccine is measured with GeneChip miRNA 4.0 Array (manufactured by Thermo Fisher Scientific), the detection signal of gga-miR-22-3p is less than 207.973, e.g., less than 84.951, and the detection signal of gga-miR-29b-1-5p is less than 5.252, e.g., less than 1.881.

It can be deemed that the inactivated whole influenza virus particle vaccine with contents of gga-miR-22-3p and gga-miR-29b-1-5p reduced to less than 50 ng/administration, more preferably 2.5 ng/administration, is a highly safe inactivated whole influenza virus particle vaccine with reduced pyrogenic activity.

This means that the pyrogenic activity of an inactivated whole influenza virus particle vaccine can be evaluated by using the contents of gga-miR-22-3p and gga-miR-29b-1-5p in the vaccine as an indicator.

Thus, according to the method of the present invention, an inactivated whole influenza virus particle vaccine with reduced pyrogenic activity can be obtained. The inactivated whole influenza virus particle vaccine may further contain a pharmaceutically acceptable carrier, in addition to the whole influenza virus particle. Examples of the carrier include carriers that are usually used in production of vaccines, specifically, a buffer, an emulsifier, a preservative (e.g., thimerosal), an isotonicity agent, a pH adjuster, an inactivating agent (e.g., formalin or beta propiolactone), and an adjuvant (e.g., aluminum hydroxide gel).

The dosage form of the inactivated whole influenza virus particle vaccine of the present invention may be, for example, a liquid, a lyophilized powder, a capsule, or a tablet.

The route of administration of the inactivated whole influenza virus particle vaccine of the present invention may be, for example, subcutaneous, intramuscular, intradermal, intranasal, sublingual, or oral administration, and the administration method may be, for example, an administration method using a syringe, a microneedle, a syringe equipped with a microneedle, a transdermal patch, or a spray.

### Examples

The present invention will now be more specifically described by Examples, but is not limited thereto. Reference Example 1: Isolation of extracellular vesicles included in uninfected egg and inactivated whole particle vaccine

B/Phuket/3073/2013 strain was inoculated in the chorioallantoic membrane inside cavity of a 12-day old embryonated chicken egg and was cultured for 2 days, and then the chorioallantoic fluid was collected. The collected chorioallantoic fluid was clarified by filter filtration and was then adsorbed to sulfuric acid barium salt, followed by elution with a 12% sodium citrate solution to collect the influenza virus. The collected virus was further replaced with 6.7 mM phosphate buffered saline (pH 7.2) by ultrafiltration, and after the buffer replacement, a fraction containing the influenza virus was collected and thereby purified by sucrose density gradient centrifugation. An inactivating agent, beta propiolactone, was added to this purified influenza virus at a final concentration of 0.05%, followed by reaction at 4°C for 24 hours to inactivate the infectiousness of the influenza virus. After this inactivation reaction, the buffer was replaced with 6.7 mM phosphate buffered saline (pH 7.2) containing 1 w/w% sucrose by ultrafiltration (MWCO: 100,000), and this was defined as an inactivated whole particle vaccine (17BY-BPL171129S, BYBPL170905, BYBPL180726, and BYBPL190425).

The inactivated whole particle vaccines (17BYBPL171129S, BYBPL170905, BYBPL180726, and BYBPL190425) prepared as described above were respectively overlaid on a 6% to 18% Iodexanol density gradient solution, followed by centrifugation at 100,600 × g at 4°C for 1.5 hours. After the centrifugation, the upper layer was separated into 11 fractions, and the individual fractions were pooled. The fractions were added to the respective centrifugal tubes and were each diluted about two-fold with 6.7 mM phosphate buffered saline (pH 7.2). The diluted fraction was centrifuged at 141,400 × g at 4°C for 3 hours, and 6.7 mM phosphate buffered saline (pH 7.2) was added to the precipitate obtained by centrifugation to obtain a suspension. This suspension was further centrifuged at 141,400 × g at 4°C for 3 hours, the supernatant after centrifugation was discarded, and the precipitate was suspended in 6.7 mM phosphate buffered saline (pH 7.2) to obtain an extracellular vesicle suspension.

Chorioallantoic fluid was collected from each of 288 12-day old embryonated chicken eggs cooled at 4°C for 1 hour or more with a disposable injection needle and a syringe (manufactured by Terumo Corporation). The collected chorioallantoic fluid was pooled and was centrifuged at 300 × g at 4°C for 10 minutes, and the obtained supernatant was further centrifuged with an ultracentrifuge (manufactured by Koki Holdings Co., Ltd.) at 100,000 × g at 4°C for 2 hours. The precipitate after the centrifugation was suspended in 6.7 mM phosphate buffered saline (pH 7.2). The suspension was overlaid on 6.7 mM phosphate buffered saline (pH 7.2) containing 25 w/w% sucrose, followed by centrifugation at 100,000 × g at 4°C for 16 hours. After the centrifugation, the upper layer was discarded, leaving 2 mL of the lower layer, the precipitate was suspended in the solution of the remaining lower layer, the suspension was diluted with 6.7 mM phosphate buffered saline (pH 7.2), followed by centrifugation at 100,000 × g at 4°C for 6 hours. The precipitate obtained by centrifugation was suspended in 6.7 mM phosphate buffered saline (pH 7.2) to obtain a noninfected egg-derived extracellular vesicle suspension (UIE EVs).

7 µL of the extracellular vesicle suspension prepared as described above and 3 µL of a fixing solution (3.3% paraformaldehyde/3.3% glutaraldehyde) were mixed and left to stand at room temperature for 5 minutes. After standing, 5 µL of the fixed extracellular vesicle suspension was added dropwise to a TEM electron microscope grid with a Formvar supporting film (manufactured by Okenshoji Co., Ltd.), followed by being left to stand for 5 minutes. Subsequently, the excessive extracellular vesicle suspension on the grid was absorbed by a paper filter, and negative staining was performed by dropping 5 µL of 2% phosphotungstic acid staining solution. The stained sample was observed and photographed with a transmission electron microscope (manufactured by JEOL Ltd.).

Consequently, as shown in Figure 1, it was confirmed that the inactivated whole particle vaccine included four types of extracellular vesicles: incomplete particles with a low spike density on the particle surfaces (EV1, Figure 1A), exosome-like particles with few surface antigens (EV2, Figure 1B), incomplete particles into which staining solution flowed (EV3, Figure 1C), and exosome-like particles with many surface antigens (EV4, Figure 1D). In addition, it was revealed that the virus particles obtained by Iodexanol density gradient centrifugation were contaminated with few extracellular vesicles (Figure 1E). It was believed that the virus particles can be used as an inactivated whole particle vaccine with extracellular vesicles removed.

### Reference Example 2: Analysis of microRNAs included in extracellular vesicle

RNA was extracted from each extracellular vesicle isolated in Reference Example 1 using miRNeasy Mini Kit (manufactured by QIAGEN). The extracted RNA was subjected to quality analysis with Nano Drop ND-1000 (manufactured by Thermo Fisher Scientific) and Bioanalyzer (manufactured by Agilent Technologies, Inc.) and then to comprehensive microRNA analysis by GeneChip miRNA 4.0 Array (manufactured by Thermo Fisher Scientific). As a result, 145 avian-derived microRNAs, 201 avian-derived microRNAs, 251 avian-derived microRNAs, 218 avian-derived microRNAs, and 328 avian-derived microRNAs were detected in EV1, EV2, EV3, EV4, and UIE EVs, respectively.

In order to select microRNAs which are induced by influenza viral infection during the vaccine preparation from the detected microRNAs, the analysis was performed by two analysis methods, i.e., analysis method <1>: selection of microRNAs detected only in EV1 to EV4, and analysis method <2>: selection of microRNAs with a signal value increased by 5 times or more compared to the signal value of UIE EVs from the microRNAs detected in UIE EVs and EV1 to EV4. In the analysis method <2>, since the amounts of loaded RNA at the time of microarray analysis were different, the obtained signals were corrected depending on the amounts loaded.

Table 1 shows microRNAs selected by the analysis method <1>. In the analysis method <1>, 60 microRNAs were selected.

**[Table 1]**

| Transcript ID(Array Design) | Transcript ID(Array Design) |
|---|---|
| gga-miR-1a-3p | gga-miR-1354 |
| gga-miR-107-5p | gga-miR-1679 |
| gga-miR-1b-5p | gga-miR-1732 |
| gga-miR-101-1-5p | gga-miR-365b-5p |
| gga-miR-1565 | gga-miR-6603-3p |
| gga-miR-1630 | gga-miR-7452-5p |
| gga-miR-1658-5p | gga-miR-155 |
| gga-miR-1711 | gga-miR-20a-3p |
| gga-miR-1739 | gga-miR-217-3p |
| gga-miR-1750 | gga-miR-133b |
| gga-miR-1784-5p | gga-miR-138-2-3p |
| gga-miR-2188-3p | gga-miR-551-5p |
| gga-miR-6547-3p | gga-miR-1641 |
| gga-miR-6558-3p | gga-miR-1693 |
| gga-miR-3594-5p | gga-miR-449c-5p |
| gga-miR-6572-5p | gga-miR-1741 |
| gga-miR-6591-3p | gga-miR-1761 |
| gga-miR-6612-5p | gga-miR-1794 |
| gga-miR-6641-3p | gga-miR-6582-5p |
| gga-miR-6672-3p | gga-miR-6585-5p |
| gga-miR-6516-3p | gga-miR-101-3p |
| gga-miR-6622-3p | gga-miR-302b-3p |
| gga-miR-7451-5p | gga-miR-1416-5p |
| gga-miR-7462-3p | gga-miR-22-5p |
| gga-miR-7473-3p | gga-miR-1689-5p |
| gga-miR-7483-5p | gga-miR-1722-5p |
| gga-miR-16-2-3p | gga-miR-1786 |
| gga-miR-146a-3p | gga-miR-6579-3p |
| gga-miR-124b | gga-miR-6610-3p |
| gga-miR-302b-5p | gga-miR-6599-5p |

Table 2 shows microRNAs selected by the analysis method <2>. In the analysis method <2>, 39 microRNAs were selected, and 99 microRNAs were selected in combination with the analysis method <1>.

**[Table 2]**

| Transcript ID(Array Design) | Transcript ID(Array Design) |
|---|---|
| gga-miR-29b-1-5p | gga-miR-1716 |
| gga-miR-222a | gga-miR-1768 |
| gga-miR-92-5p | gga-miR-1788-3p |
| gga-miR-30b-3p | gga-miR-101-2-5p |
| gga-miR-30c-2-3p | gga-miR-130c-3p |
| gga-miR-184-3p | gga-miR-193a-5p |
| gga-miR-146a-5p | gga-miR-2126 |
| gga-miR-130a-3p | gga-miR-2131-5p |
| gga-miR-34a-5p | gga-miR-2954 |
| gga-miR-31-5p | gga-miR-6560-3p |
| gga-miR-455-3p | gga-miR-6580-5p |
| gga-miR-22-3p | gga-miR-6590-3p |
| gga-miR-1456-5p | gga-miR-6642-5p |
| gga-miR-1462-5p | gga-miR-6642-3p |
| gga-miR-1466 | gga-miR-6695-5p |
| gga-miR-1563 | gga-miR-7439-3p |
| gga-miR-1581 | gga-miR-7442-5p |
| gga-miR-1594 | gga-miR-7471-5p |
| gga-miR-1599 | gga-miR-7471-3p |
| gga-miR-1668-5p | |

The selected 99 microRNAs were subjected to homology analysis with human and mouse microRNAs using miRBase Release 22.1 (URL: https://mirbase.org/). 64 types of human microRNAs and 70 types of mouse microRNAs were recognized to have high homology. The results of the homology analysis are shown in Tables 3 and 4.

**[Table 3]**

| Chicken | Human | Strand | Chicken | Human | Strand |
|---|---|---|---|---|---|
| gga-miR-1a-3p | hsa-miR-1-3p | + | gga-miR-302b-3p | hsa-miR-302b-3p | + |
| gga-miR-107-5p | hsa-miR-103a-2-5p | + | | hsa-miR-302c-3p | + |
| | hsa-miR-103a-1-5p | + | | hsa-miR-302a-3p | + |
| gga-miR-1b-5p | hsa-miR-1-5p | + | | hsa-miR-302d-3p | + |
| gga-miR-101-1-5p | hsa-miR-101-2-5p | + | | hsa-miR-302e | + |
| gga-miR-1630 | hsa-miR-4496 | + | | hsa-miR-302f | + |
| gga-miR-1658-5p | hsa-miR-2681-5p | + | | hsa-miR-302d-5p | - |
| gga-miR-1711 | hsa-miR-143-5p | + | | hsa-miR-302a-5p | - |
| | hsa-miR-143-3p | - | | hsa-miR-302b-5p | - |
| gga-miR-6516-3p | hsa-miR-6516-3p | + | gga-miR-29b-1-5p | hsa-miR-29b-1-5p | + |
| gga-miR-7451-5p | hsa-miR-628-3p | + | | hsa-miR-29b-2-5p | + |
| gga-miR-7483-5p | hsa-miR-138-5p | + | gga-miR-222a | hsa-miR-222-3p | + |
| gga-miR-16-2-3p | hsa-miR-16-2-3p | + | gga-miR-92-5p | hsa-miR-92a-1-5p | + |
| | hsa-miR-195-5p | - | gga-miR-30b-3p | hsa-miR-30b-3p | + |
| gga-miR-146a-3p | hsa-miR-146a-5p | - | | hsa-miR-30c-1-3p | + |
| gga-miR-124b | hsa-miR-124-3p | + | | hsa-miR-30c-2-3p | + |
| gga-miR-302b-5p | hsa-miR-302b-5p | + | gga-miR-30c-2-3p | hsa-miR-30c-2-3p | + |
| | hsa-miR-302d-5p | + | | hsa-miR-30c-1-3p | + |
| | hsa-miR-302a-5p | + | | hsa-miR-30b-3p | + |
| | hsa-miR-302c-5p | + | gga-miR-184-3p | hsa-miR-184 | + |
| gga-miR-365b-5p | hsa-miR-365a-3p | - | gga-miR-146a-5p | hsa-miR-146a-5p | + |
| | hsa-miR-365b-3p | - | | hsa-miR-146b-5p | + |
| gga-miR-155 | hsa-miR-155-5p | + | gga-miR-130a-3p | hsa-miR-130a-3p | + |
| gga-miR-20a-3p | hsa-miR-20a-3p | + | gga-miR-34a-5p | hsa-miR-34a-5p | + |
| | hsa-miR-20a-5p | - | gga-miR-31-5p | hsa-miR-31-5p | + |
| gga-miR-217-3p | hsa-miR-217-3p | + | gga-miR-455-3p | hsa-miR-455-3p | + |
| | hsa-miR-217-5p | - | gga-miR-22-3p | hsa-miR-22-3p | + |
| gga-miR-133b | hsa-miR-133b | + | gga-miR-1466 | hsa-miR-4763-3p | + |
| gga-miR-138-2-3p | hsa-miR-138-2-3p | + | gga-miR-1668-5p | hsa-miR-5008-3p | - |
| | hsa-miR-138-1-3p | + | | hsa-miR-619-5p | + |
| gga-miR-551-5p | hsa-miR-551b-5p | + | gga-miR-1716 | hsa-miR-194-3p | + |
| | hsa-miR-551a | - | gga-miR-101-2-5p | hsa-miR-101-5p | + |
| gga-miR-449c-5p | hsa-miR-4793-3p | + | | hsa-miR-101-3p | - |
| | hsa-miR-2278 | + | gga-miR-130c-3p | hsa-miR-130a-3p | + |
| gga-miR-101-3p | hsa-miR-101-3p | + | | hsa-miR-130b-3p | + |
| | hsa-miR-101-5p | - | gga-miR-193a-5p | hsa-miR-193a-5p | + |
| gga-miR-22-5p | hsa-miR-22-5p | + | | hsa-miR-193b-5p | + |

**[Table 4]**

| Chicken | Mouse | Strand | Chicken | Mouse | Strand |
|---|---|---|---|---|---|
| gga-miR-1a-3p | mmu-miR-1a-3p | + | gga-miR-302b-3p | mmu-miR-302b-3p | + |
| | mmu-miR-1b-5p | - | | mmu-miR-302a-3p | + |
| gga-miR-107-5p | mmu-miR-107-5p | + | | mmu-miR-302d-3p | + |
| | mmu-miR-103-2-5p | + | | mmu-miR-302c-3p | + |
| | mmu-miR-103-1-5p | + | | mmu-miR-302d-5p | - |
| gga-miR-1b-5p | mmu-miR-1a-1-5p | + | gga-miR-6599-5p | mmu-miR-6935-5p | + |
| | mmu-miR-1a-2-5p | + | gga-miR-29b-1-5p | mmu-miR-29b-1-5p | + |
| | mmu-miR-206-5p | + | | mmu-miR-29b-2-5p | + |
| | mmu-miR-1b-3p | - | gga-miR-222a | mmu-miR-222-3p | + |
| gga-miR-101-1-5p | mmu-miR-101b-5p | + | gga-miR-92-5p | mmu-miR-92a-1-5p | + |
| gga-miR-1711 | mmu-miR-143-5p | + | gga-miR-30b-3p | mmu-miR-30b-3p | + |
| | mmu-miR-143-3p | - | | mmu-miR-30c-1-3p | + |
| gga-miR-3594-5p | mmu-miR-7226-3p | + | | mmu-miR-30c-2-3p | + |
| gga-miR-6572-5p | mmu-miR-7009-3p | + | gga-miR-30c-2-3p | mmu-miR-30c-2-3p | + |
| gga-miR-6641-3p | mmu-miR-7073-3p | - | | mmu-miR-30c-1-3p | + |
| gga-miR-6516-3p | mmu-miR-6516-3p | + | | mmu-miR-298-3p | - |
| gga-miR-7483-5p | mmu-miR-138-5p | + | gga-miR-184-3p | mmu-miR-184-3p | + |
| gga-miR-16-2-3p | mmu-miR-16-2-3p | + | gga-miR-146a-5p | mmu-miR-146a-5p | + |
| | mmu-miR-195a-5p | - | | mmu-miR-146b-5p | + |
| gga-miR-146a-3p | mmu-miR-146a-5p | - | gga-miR-130a-3p | mmu-miR-130a-3p | + |
| | mmu-miR-146b-3p | + | gga-miR-34a-5p | mmu-miR-34a-5p | + |
| gga-miR-302b-5p | mmu-miR-302b-5p | + | gga-miR-31-5p | mmu-miR-31-5p | + |
| | mmu-miR-302d-5p | + | gga-miR-455-3p | mmu-miR-455-3p | + |
| gga-miR-365b-5p | mmu-miR-365-3p | - | gga-miR-22-3p | mmu-miR-22-3p | + |
| gga-miR-155 | mmu-miR-155-5p | + | | mmu-miR-3966 | + |
| gga-miR-20a-3p | mmu-miR-20a-3p | + | gga-miR-1462-5p | mmu-miR-6407 | + |
| | mmu-miR-20a-5p | - | | mmu-miR-6908-5p | - |
| gga-miR-217-3p | mmu-miR-217-3p | + | gga-miR-1563 | mmu-miR-291a-3p | - |
| | mmu-miR-217-5p | - | gga-miR-1668-5p | mmu-miR-6970-5p | + |
| gga-miR-133b | mmu-miR-133b-3p | + | gga-miR-1716 | mmu-miR-194-2-3p | + |
| gga-miR-551-5p | mmu-miR-551b-5p | + | gga-miR-101-2-5p | mmu-miR-101a-5p | + |
| gga-miR-138-2-3p | mmu-miR-138-2-3p | + | | mmu-miR-101a-3p | - |
| | mmu-miR-138-1-3p | + | | mmu-miR-101c | - |
| gga-miR-6585-5p | mmu-miR-1912-3p | + | gga-miR-130c-3p | mmu-miR-130a-3p | + |
| gga-miR-101-3p | mmu-miR-101a-3p | + | | mmu-miR-130b-3p | + |
| | mmu-miR-101c | + | gga-miR-193a-5p | mmu-miR-193a-5p | + |
| | mmu-miR-101b-3p | + | | mmu-miR-193b-5p | + |
| | mmu-miR-101a-5p | - | gga-miR-2954 | mmu-miR-6995-5p | - |
| gga-miR-22-5p | mmu-miR-22-5p | + | gga-miR-6560-3p | mmu-miR-540-3p | - |

As shown in Table 5, microRNAs with high homology were subjected to functional analysis, and as microRNAs which enhances an immune or inflammatory response, five microRNAs: gga-miR-551-5p (GAAAUCAAGGGUGGGUAAGACCU: SEQ ID No: 5), gga-miR-22-3p (AAGCUGCCAGUUGAAGAACUGU: SEQ ID No: 2), gga-miR-1466 (GCGCUCAGGCUGGUGCUGGGGGAU: SEQ ID No: 3), gga-miR-155 (UUAAUGCUAAUCGUGAUAGGGG: SEQ ID No: 4), and gga-miR-29b-1-5p (AGCUGGUUUCAUAUGGUGGUUUAGA: SEQ ID No: 1) were selected.

**[Table 5]**

| Chicken | Human/Mouse | Role | EV1 | EV2 | EV3 | EV4 | UIE EVs |
|---|---|---|---|---|---|---|---|
| Enhancement of immune or inflammatory responses | | | | | | | |
| gga-miR-551-5p | hsa-miR-551b-5p | Enhancement of inflammatory response | | | **good** | | |
| gga-miR-22-3p | hsa-miR-22-3p | Enhancement of IL-1β, IL-6, and the like | | good | **good** | **good** | good |
| gga-miR-1466 | hsa-miR-4763-3p | Enhancement | good | good | **good** | good | good |
| gga-miR-155 | mmu-miR-155-5p | Enhancement of IL-6, TNF-α, IFN-β, and INF-y | | | **good** | **good** | |
| gga-miR-29b-1-5p | mmu-miR-29b-1-5p | Promotion of NF-kB signaling | | | **good** | good | good |

| Suppression of immune or inflammatory responses | | | | | | | |
|---|---|---|---|---|---|---|---|
| gga-miR-7451-5p | hsa-miR-628-3p | Suppression of expression of TLR3, RIG-I, and MDA-5 | **good** | | | | |
| gga-miR-7483-5p | hsa-miR-138-5p | Suppression of NF-κB | **good** | | | | |
| gga-miR-124b | hsa-miR-124-3p | Suppression of IL-11 | | **good** | **good** | | |
| gga-miR-155 | hsa-miR-155-5p | Suppression of NF-κB | | | **good** | **good** | |
| gga-miR-101-3p | hsa-miR-101-3p | Suppression of TLR2 | | | | **good** | |
| gga-miR-302b-3p | hsa-miR-302c-3p | Suppression of IL-8 | | | | **good** | |
| | hsa-miR-302d-3p | Suppression of IL-8 | | | | **good** | |
| gga-miR-222a | hsa-miR-222-3p | Suppression of IL-21 | good | good | **good** | good | good |
| gga-miR-30b-3p | hsa-miR-30c-2-3p | Suppression of NF-κB | | | **good** | **good** | good |
| gga-miR-146a-5p | hsa-miR-146a-5p | Suppression of TLR4 | | | **good** | **good** | good |
| | hsa-miR-146b-5p | Suppression of IL-17 | | | **good** | **good** | good |
| gga-miR-130a-3p | hsa-miR-130a-3p | Suppression of TNF-α | | good | **good** | **good** | good |
| gga-miR-34a-5p | hsa-miR-34a-5p | Suppression of BCR pathway | | good | **good** | **good** | good |
| gga-miR-31-5p | hsa-miR-31-5p | Suppression of inflammatory response | good | good | **good** | good | good |
| gga-miR-193a-5p | hsa-miR-193a-5p | Suppression of IL-12β | | good | **good** | **good** | good |
| gga-miR-146a-3p | mmu-miR-146b-3p | Suppression of IL-1β, IL-6, and TNF-α | | **good** | **good** | **good** | |
| gga-miR-20a-3p | mmu-miR-20a-3p | Suppression of IL-1β, IL-6, and TNF-α | | | **good** | | |
| gga-miR-302b-3p | mmu-miR-302b-3p | Suppression of IL-1β, IL-6, and TNF-α | | | | **good** | |
| | mmu-miR-302a-3p | Suppression of IL-1β, IL-6, and TNF-α | | | | **good** | |
| gga-miR-222a | mmu-miR-222-3p | Suppression of IL-6, MCP-1, and IFN-β | good | good | **good** | good | good |
| gga-miR-130a-3p | mmu-miR-130a-3p | Suppression of TNF-α | | good | **good** | **good** | good |
| gga-miR-130c-3p | mmu-miR-130a-3p | Suppression of TNF-α | | good | **good** | **good** | good |

good: Sample in which microRNA was detected
**good:**Sample exhibited a sample-specific increase or five times or more increase in signal with respect to control

### Reference Example 3: pyrogenicity test using mouse

In order to verify whether the pyrogenic activity of an inactivated whole particle vaccine (B/Phuket/3073/2013 strain) can be evaluated using a mouse, an inactivated whole particle vaccine (300 µg as protein amount) or a saline solution was intraperitoneally administered to Balb/c mice (female 17-week old, N = 8). Rectal temperature was measured before the administration, immediately after administration, and at 2, 4, 6, and 8 hours after administration. In addition, as the baseline body temperature measurement, rectal temperature was measured at the same time as above on the day before the administration. Difference in the body temperature was calculated by subtracting the baseline body temperature from the body temperature after administration to evaluate a variation in body temperature.

The baseline body temperatures in the inactivated whole particle vaccine administration group and the saline solution administration group were equal to each other and showed a similar trend (Figure 2A). In contrast, although the body temperature after administration in the saline solution administration group changed similarly to the baseline body temperature, in the inactivated whole particle vaccine administration group, the body temperature remained higher than the baseline body temperature (Figure 2B). The difference in the body temperature also remained around 0.0°C in the saline solution administration group, but in the inactivated whole particle vaccine administration group, pyrogenic reaction of 0.8°C at the maximum was observed (Figure 2C), and it was confirmed that the pyrogenic activity of an inactivated whole particle vaccine could be evaluated by a pyrogenicity test using a mouse.

### Example 1: Pyrogenic activity of extracellular vesicle-removed inactivated whole particle vaccine

In order to evaluate the pyrogenic activity of the inactivated whole particle vaccine with removed extracellular vesicle (extracellular vesicle-removed inactivated whole particle vaccine: B/Phuket/3073/2013 strain) prepared by Iodexanol density gradient centrifugation in Reference Example 1, a mouse pyrogenicity test was performed. Balb/c mice (female, 9-week old, N = 6) were each intraperitoneally given 150 µg (as total protein amount) of the inactivated whole particle vaccine or the extracellular vesicle-removed inactivated whole particle vaccine, or the saline solution as a control, and the rectal temperature was measured immediately after administration and at 2, 4, and 6 hours after administration. In addition, as the baseline body temperature measurement, rectal temperature was measured at the same time as above on the day before the administration. The difference in the body temperature was calculated by subtracting the baseline body temperature from the body temperature after administration to evaluate a variation in body temperature.

The baseline body temperatures were similar in all groups and showed a similar trend (Figure 3-1A). In the inactivated whole particle vaccine administration group, the body temperature after administration remained higher than the baseline body temperature (Figure 3-1B), and the difference in the body temperature also remained higher than 0.5°C from 2 hours after administration (Figure 3-1C). Thus, the pyrogenic reaction by the inactivated whole particle vaccine administration was observed. In contrast, in the extracellular vesicle-removed inactivated whole particle vaccine administration group, the body temperature after administration remained to be around the baseline body temperature (Figure 3-1B), and the difference in the body temperature also remained around 0.0°C (Figure 3-1C). Accordingly, it was believed that the pyrogenic activity of the inactivated whole particle vaccine was decreased by removing extracellular vesicles. The difference in the body temperature of each individual at 2 hours after administration was verified, the average value was 0.6°C in the inactivated whole particle vaccine administration group, 0.1°C in the extracellular vesicle-removed inactivated whole particle vaccine administration group, and 0.0°C in the saline solution administration group. Thus, the pyrogenic reaction was observed only in the inactivated whole particle vaccine administration group, and the trend was also observed in the distribution of individuals. Therefore, it was demonstrated that the pyrogenic reaction in mice could be evaluated at 2 hours after administration (Figure 3-2).

RNAs extracted from the extracellular vesicle-removed inactivated whole particle vaccine (B/Phuket/3073/2013 strain) used in this test and the extracellular vesicles (EV1 to EV4) and the uninfected egg-derived extracellular vesicle suspension (UIE EVs) prepared in Reference Example 1 were subjected to microarray analysis by GeneChip miRNA 4.0 Array (manufactured by Thermo Fisher Scientific). The signal values of five types of microRNAs which were suspected to be involved in pyrexia are shown in Table 6. In the microarray chip used, a 2-fold change can be considered as a significant change, and the content of every microRNA in the extracellular vesicle-removed inactivated whole particle vaccine was less than a half compared to the EVs fraction which showed the highest value. Thus, a significant decrease was confirmed.

**[Table 6]**

| Item | EV1 | EV2 | EV3 | EV4 | UIE EVs | Extracellular vesicle-removed inactivated whole particle vaccine |
|---|---|---|---|---|---|---|
| gga-miR-551-5p | 4.019* | 3.521* | 7.801 | 3.375* | 1.483* | 1.331* |
| gga-miR-22-3p | 3.923* | 137.327 | 415.946 | 380.796 | 61.708 | 84.951 |
| gga-miR-1466 | 19.613 | 14.957 | 44.923 | 15.971 | 6.539 | 1.990 |
| gga-miR-155 | 4.019* | 3.521* | 4.695 | 2.817 | 1.374* | 1.408* |
| gga-miR-29b-1-5p | 2.993* | 2.603* | 10.504 | 4.904 | 1.771 | 1.881* |

| | | | | | | |
|---|---|---|---|---|---|---|
| *:Below detection limit | | | | | | |

### Reference Example 4: Setting of microRNA dosage

For evaluating the pyrogenic activity of a microRNA, it is necessary to verify the reversion to pyrogenic reaction by co-administration of the extracellular vesicle-removed inactivated whole particle vaccine (B/Phuket/3073/2013 strain) with reduced pyrogenic activity prepared in Reference Example 1 and a microRNA which was suspected to be involved in pyrogenic activity. However, it was considered that there is a possibility of causing an immune response by recognition of a microRNA, which is a short-chain RNA, itself by a nucleic acid receptor such as TLR and expressing sequence-independent pyrogenic reaction. Accordingly, it was attempted to determine a microRNA dosage that would not affect the pyrogenic reaction.

5000 ng, 500 ng, 50 ng, or 5 ng of a microRNA was intraperitoneally administrated to Balb/c mice (female, 10-week old, N = 6). The microRNA used was gga-miR-2188-5p having low homology with human and mouse microRNAs and believed not to affect immune and inflammatory responses. The RNA was embedded in Invivofectamine 3.0 Reagent (manufactured by Thermo Fisher Scientific) and was then administered. The rectal temperature was measured immediately after the administration and at 2, 4, and 6 hours after administration. In addition, as the baseline body temperature measurement, rectal temperature was measured at the same time as above on the day before the administration. The difference in the body temperature was calculated by subtracting the baseline body temperature from the body temperature after administration to evaluate a variation in body temperature.

The baseline body temperatures were similar in all groups and showed a similar trend (Figure 4A), the body temperatures after the administration were also equal to the baseline body temperatures in every group and showed a similar trend (Figure 4B). The difference in the body temperature of each individual at 2 hours after the administration distributed around 0.0°C in every group (Figure 4C), and no clear trend by dosage of the microRNA was recognized. Accordingly, it was believed that the pyrogenic activity of the microRNA itself is not expressed within the range of 5 to 5000 ng.

### Example 2: Verification of pyrogenic activity of microRNA present in inactivated whole particle vaccine

In order to verify whether five types of microRNAs, which were believed to promote an immune or inflammatory response in Reference Example 2, affect pyrogenic activity or not, the pyrogenic reaction when the extracellular vesicle-removed inactivated whole particle vaccine (B/Phuket/3073/2013 strain) prepared in Reference Example 1 was co-administered with the respective microRNAs was evaluated.

An extracellular vesicle-removed inactivated whole particle vaccine (150 µg as protein amount) only or a vaccine which was a mixture of extracellular vesicle-removed inactivated whole particle vaccine (150 µg as protein amount) and a microRNA (500 ng of gga-miR-551-5p, gga-miR-22-3p, gga-miR-1466, gga-miR-155, or gga-miR-29b-1-5p) was intraperitoneally administrated to Balb/c mice (female, 12-week old, N = 8). The microRNA was embedded in Invivofectamine 3.0 Reagent (manufactured by Thermo Fisher Scientific) and was then administered. The rectal temperature was measured immediately after the administration and at 2, 4, and 6 hours after the administration, and as the baseline body temperature measurement, the rectal temperature was measured at the same time as above on the day before the administration. The difference in the body temperature was calculated by subtracting the baseline body temperature from the body temperature after administration to evaluate a variation in body temperature.

As shown in Figure 5, the difference in the body temperature at 2 hours after the administration was 0.21°C when only the extracellular vesicle-removed inactivated whole particle vaccine was administered, while the difference in the body temperature was 0.56°C in co-administration with gga-miR-29b-1-5p and 0.48°C in co-administration with gga-miR-22-3p. Thus, the increase in the difference in the body temperature was observed. Accordingly, it is evident that gga-miR-29b-1-5p and gga-miR-22-3p enhance the pyrogenic reaction of the inactivated whole particle vaccine, and it is evident that a vaccine not containing these two microRNAs will be a safe vaccine with reduced pyrogenic activity.

## Claims

1. A method for reducing pyrogenic activity of an inactivated whole influenza virus particle vaccine, the method comprising a step of reducing the content of an avian-derived microRNA in a virus solution containing a whole influenza virus particle collected from an embryonated chicken egg in a method for preparing the vaccine using an embryonated chicken egg method.

2. The method according to Claim 1, wherein the contents of at least gga-miR-22-3p and gga-miR-29b-1-5p are reduced.

3. An influenza virus vaccine according to Claim 1 or 2, wherein the whole influenza virus particle contains one or both of a type A influenza virus strain and a type B influenza virus strain.

4. A method for evaluating pyrogenic activity of an inactivated whole influenza virus particle vaccine using an embryonated chicken egg method, comprising a step of measuring the contents of gga-miR-22-3p and gga-miR-29b-1-5p in the vaccine.

5. An inactivated whole influenza virus particle vaccine prepared by an embryonated chicken egg method and having reduced contents of gga-miR-22-3p and gga-miR-29b-1-5p.
